# EUROPEAN PATENT APPLICATION

(11) **EP 2 179 722 A1**
(43) Date of publication of application: **28.04.2010**
(21) Application number: 08018659.6
(22) Date of filing: 24.10.2008
(51) Int. Cl.: A61K 9/00, A61K 36/00

(54) **Topical formation for preventing sexual transmission of viral infection**

(71) Applicant: Heinrich-Pette-Institut für experimentelle Virologie und Immunologie an der Universität Hamburg, 20251 Hamburg (DE)
(72) Inventor: Hauber, Ilona, 22527 Hamburg (DE); Hauber, Joachim, 20535 Hamburg (DE); Hohenberg, Heinrich, 22767 Hamburg (DE)
(74) Representative: UEXKÜLL & STOLBERG

(57) **Abstract**

The present invention relates to the prevention of sexual transmission of viral infection, in particular, HIV infection. The invention provides a pharmaceutical composition for topical administration for prevention of sexual transmission of viral infection, wherein the composition comprises at least one compound from the class of catechins, such as epigallo-catechin-gallate (EGCG). The invention also relates to the use of at least one compound from the class of catechins for the preparation of a pharmaceutical composition formulated for topical administration for prevention of sexual transmission of viral infection. The invention further provides a method for preventing sexual transmission of viral infection.

## Description

The present invention relates to the prevention of sexual transmission of viral infection, in particular, HIV infection. The invention provides a pharmaceutical composition for topical administration for prevention of sexual transmission of viral infection, wherein the composition comprises at least one compound from the class of catechins. The invention further provides the use of at least one compound from the class of catechins for the preparation of a pharmaceutical composition formulated for topical administration for prevention of sexual transmission of viral infection. The invention also relates to a method for preventing sexual transmission of viral infection.

On the global scale, the HIV-1 epidemic is primarily driven by heterosexual transmission, accounting for the vast majority of the currently 33 million people infected with this lethal pathogen (1). While more than 96% of new infections occur in low and middle income countries, it is expected that the successful clinical development of antiretroviral drug-based microbicides may not only significantly slow sexual transmission of HIV-1, but may also provide a simple and affordable prevention method, particularly for application in resource-poor settings (reviewed in (2, 3)).

In particular, it would be desirable to provide a microbicide for topical application to prevent sexual transmission of viral diseases. This would render it possible, e.g., for women to protect themselves in the context of sexual intercourse, in case use of condoms is not possible or not desired for some reason, or in addition to the use of condoms. The first generation of microbizides against HIV that was clinically tested, e.g., Carraguard, Savvy, Ushercell and nonoxynol-9), unfortunately turned out to be ineffective. A second generation of microbizides, based on antiretroviral substances like reverse transcriptase inhibitors, is presently being tested. However, these might not solve all problems, because it is well known that the development of resistance to specific antiretroviral drugs is common. Such drugs would be specific for antiretroviral therapy, and they can only exert their effects after infection of cells has occurred. Furthermore, such substances are expensive and their cost might lead to problems of availability at least in low and middle income countries. Alternative topical therapeutics able to prevent sexual transmission of viral diseases would thus be desirable. This technical field presently faces various technical challenges (8).

During the process of identifying natural agents that interfere with HIV-1 replication, Münch and coworkers recently identified a peptide fraction in human semen (SE) that consistently enhanced HIV-1 infection (4). The major enhancing activity was correlated with the presence of a peptide derived from the internal region of prostatic acidic phosphatase (PAP) (4), a protein that is produced by the prostatic gland and secreted in large quantities (1-2 mg/ml) into SE (5). Interestingly, the ability of this peptide, corresponding to PAP amino acid residues 248-286 (PAP248-286), to boost infectivity of a broad range of HIV-1 strains, including X4-, R5-, and dual-tropic isolates as well as group M and O strains, depended on its unexpected capacity to form β-sheet-rich amyloid fibrils (4). Although their exact mode of action is largely unknown, these fibrils, termed semen-derived enhancer of virus infection (SEVI), apparently capture virions and attach them to the surface of target cells, thereby enhancing receptor-mediated virus-host cell fusion (discussed in (6). Thus, semen-derived fibril-forming PAP fragments may strongly promote HIV transmission during sexual intercourse, particularly when low quantities of infectious virus are available to cross the mucosal barrier (reviewed in (2, 7)).

Many highly diverse proteins are able to self-assemble into amyloid fibrils (9, 10), a process that is frequently associated with serious neurodegenerative disorders such as, for example, Parkinson's disease and Alzheimer's disease (11, 12). In a recent study it has been investigated whether small molecule inhibitors can interfere with amyloid formation and, in particular, it has been demonstrated that the polyphenol epigallocatechin-gallate (EGCG) and other compounds from the class of catechins efficiently inhibit amyloid fibrillogenesis (13, WO 2005/077343, US 2002/0151506, US 2002/0086067) .

EGCG is the major catechin in green tea and has been ascribed to exert various anti-tumorigenic, anti-oxidative, antibacterial and antiviral effects (for reviews see (14-16)).

Antiviral effects against papilloma virus are disclosed in US 5,795,911 and US 5,968,973. Effects on HIV, e.g., HIV replication are described in (19) and (20), leading to proposals to use catechins as systemic therapeutics for treatment of HIV.

US 2002/0151506 discloses that effects of catechins on fibril formation can vary, in particular, they can depend on the type of amyloid protein.

Ehrnhoefer et al. (13) teach that the polyphenol (-)epigallocatechin gallate (EGCG) efficiently inhibits the fibrillogenesis of both α-synuclein and amyloid-β by directly binding to the natively unfolded polypeptides and preventing their conversion into toxic, on-pathway aggregation intermediates. Instead of β-sheet-rich amyloid, the formation of unstructured, non-toxic α-synuclein and amyloid-β oligomers of a new type is promoted.

The prior art thus allowed no conclusions on the potential of catechins, in particular EGCG, to target PAP248-286-derived fibrils and to interfere with semen-mediated enhancement of viral infection. Specifically, it was unknown if catechins are able to prevent SEVI formation or to interfere with SEVI fibrils that have already formed, and if any alternative oligomer of the PAP248-286 peptide that might be formed after interaction with the catechin would enhance or inhibit viral infection.

In light of the state of the art, the skilled person was thus faced with the problem of providing a pharmaceutical composition for topical administration for preventing sexual transmission of viral infection. Preferably, such a composition should be affordable, effective, even in the light of development of resistance, as well as easy to use.

This problem is solved by the present invention. The inventors demonstrated that catechins, such as EGCG, suppress the infectivity-enhancing effect of human semen by interfering with their formation and degrading semen-derived amyloid fibrils. The invention thus provides a pharmaceutical composition for topical administration for prevention of sexual transmission of viral infection, wherein the composition comprises at least one compound from the class of catechins. The present invention also provides the use of at least one compound from the class of catechins for the preparation of a pharmaceutical composition formulated for topical administration for prevention of sexual transmission of viral infection.

In the context of the invention, prevention of sexual transmission of viral infection means that the risk of infection upon sexual contact is significantly reduced, preferably, by a factor of about 50%, about 80%, about 90% or about 99%. The composition of the present invention may thus be used for preventing viral infection.

The invention also relates to a method for preventing sexual transmission of viral infection, comprising topically administering a composition comprising at least one compound from the class of catechins.

Catechins are polyphenolic antioxidant plant metabolites belonging to the family of flavan-3-ols. These compounds can be derived from tea plants of the *Theaceae* species, in particular *Camellia sinensis,* and are also found in some cocoas and chocolates (made from the seeds of *Theobroma cacao*). Spotted knapweed (*Centaurea stoebe*) also contains catechins.

In the context of the present invention, a compound from the class of catechins can be selected from the group consisting of catechin (C), catechin-gallate (CG), gallocatechin (GC), gallocatechin-gallate (GCG), epicatechin (EC), epicatechin-gallate (ECG), epigallocatechin (EGC), epigallocatechin-gallate (EGCG), and derivatives thereof which are capable of disrupting preformed SEVI (semen-derived enhancer of virus infection) fibrils. The (+) or (-) isomers of the compounds can be used, however, the (-) isomers are preferred.

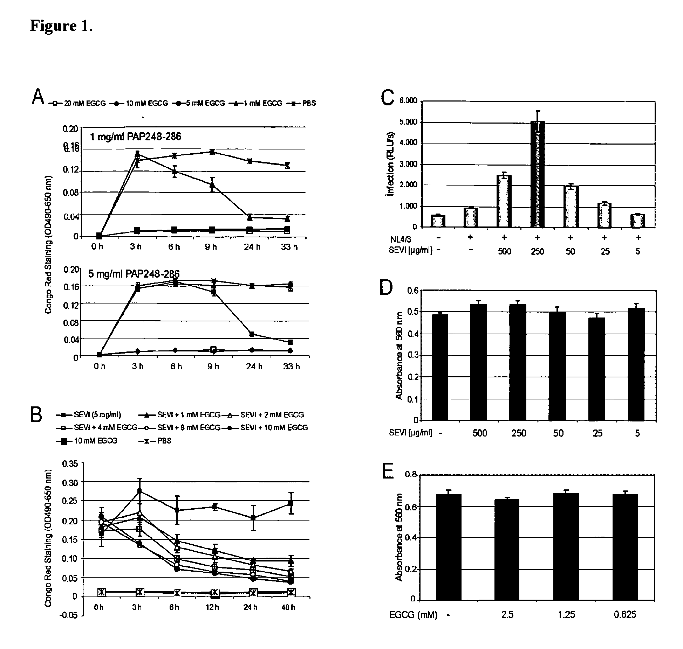

Derivatives of the catechins, e.g., of EGCG, can be substituted with lower alkyl (C1-C6) groups, in particular, Methyl or ethyl groups, or with additional hydroxyl groups. Esters of catechins (e.g., C, GC, EC, EGC) with other acids instead of gallic acid, e.g., with 3,4-dihydroxybencoic acid or 3,5-dihydroxybencoic acid can also be useful in the context of the invention. The efficacy of such catechin compounds can be tested, e.g., in the assays described below, in particular, in the assay for disruption of preformed SEVI shown in Fig. 1B.

In the context of the present invention, preferably, the compound is epigallocatechin-gallate (EGCG), in particular (-)EGCG.

For preparation of the composition according to the present invention, the compound may be derived from tea, such as green tea, in particular from tea leaves or an extract thereof (GTE; green tea extract). Standardized green tea extracts, e.g., standardized to 50% polyphenols, are available commercially, and preparation of catechins, both by isolation or synthesis, is known in the state of the art (e.g., US 2002/0151506, WO 2005/077343, (13)). In one embodiment of the invention, the composition comprises green tea (*Camellia sinensis*) extract or white tea extract. Compositions that may be employed, are, e.g., Polyphenon E^{™}, produced by Mitsui Norin Co., which may comprise, e.g.: (-)EC 10.8%, (-)EGC 9.2%, (-)ECG 6.5%, (-)EGCG 54.8%, and (-)GCG 4.0% or Polyphenon 100^{™}, produced by Mitsui Norin Co., which may comprise, e.g.: (+)GC 1.44%, (-)EC 5.81%, (-)EGC 17.57%, (-)ECG 12.51%, (-)EGCG 53.90.

The composition may be formulated as an ointment, cream, gel, suppository, liquid for vaginal irrigation, intravaginal ring or concentrate for preparation of the same. For example, the topical administration forms disclosed in US 5,795,911 or 5,968,973 may be used in the context of the present invention. Vaseline, e.g., may be used as a basis for an ointment.

In one embodiment of the invention, the composition is formulated for topical administration to the vagina of a woman before and/or after sexual intercourse. Other methods of topical administration are possible, such as administration to the penis (e.g., formulated as a lubricant), and may also depend on sexual practices.

Münch and coworkers note that SEVI fibrils are highly stable, and could potentially facilitate HIV infection in the genital tract for relatively long time periods. Therefore, the composition of the present invention may still have an effect in reducing the risk of sexual transmission if applied directly (up to one hour) after intercourse, after about one hour, after about 3 hours, after about 6 hours or after about 12 or after about 24 hours after sexual intercourse. A single application may be sufficient to significantly reduce the risk of infection. Preferably, it is applied directly before and/or after sexual intercourse. Sustained release forms may be used.

The composition for use in the present invention comprises an amount of the catechins compound effective to prevent sexual transmission of viral infections, in particular, to significantly reduce the risk of their sexual transmission. In one embodiment of the invention, the composition is formulated to achieve intravaginal concentrations of the compound of about 0.1 mM or more, about 0.25 mM or more, about 0.5 mM or more, about 1 mM or more or about 2.5 mM or more. The inventors have shown that such concentrations may be effective to significantly reduce infection rates. In one embodiment, the composition, e.g., an ointment, comprises about 2-20% by weight of catechins, preferably, about 5-18% or about 10-15%. A suppository might contain about 50-500 mg/capsule, preferably, about 200-300 mg/capsule or about 250 mg/capsule.

The composition may also comprise antiviral or antiretroviral agents, e.g. reverse transcriptase inhibitors or proteinase inhibitors.

According to the invention, the composition may be used for preventing viral infection, in particular, for preventing sexual transmission of a (sexually transmissible) viral infection. In a preferred embodiment, the infection is retroviral infection. However, as the agent does not target a virus mechanism of infection, but a host mechanism responsible for enhancing infectivity of different viruses, the compositions of the invention are also effective against infection with a DNA containing virus. For example, the infection may be with HIV (Human immunodeficiency virus), in particular, HIV-1 or HIV-2, HTLV (Human T-Lymphotropic Virus, in particular, HTLV-1 or HTLV-2), Hepatitis B, Hepatitis C, Human papilloma virus (HPV) and/or Herpes-simplex virus (HSV) and human Herpesvirus 8 (HHV8).

While previous publications stated that catechins might be effective in treatment of some of these viruses, the inventors could demonstrate that effects of the catechins on virus infection are significantly higher in the presence of SEVI. In fact, no effects of EGCG on HIV infection could be shown in the absence of SEVI. This demonstrates that the topical use of catechins provided by the present invention, which was not considered before, can be a much more efficient means of preventing infection than systemic administration which was suggested by the prior art.

It is noted that the present invention allows preventing infection with viruses, even in case these viruses have developed resistance against other antiviral agents, and it is based on a mechanism that cannot easily be circumvented by viral mutations. Thus, the composition can also be used to prevent sexual transmission of multi-resistant virus strains.

In the following examples, the invention is further illustrated. The scope of the invention is not intended to be limited to the examples.

All literature cited herein is fully incorporated herein by reference.

### FIGURE LEGEND

**Figure 1****. EGCG targets synthetic PAP248-286-derived amyloid fibrils**. (A) Amyloid fibrils were formed by agitation of the fresh PAP248-286 solutions (1 mg/ml and 5 mg/ml) at 37°C. Fibrillar aggregates were exposed to various concentrations of EGCG (□, 20 mM; •, 10 mM; ■, 5 mM; and ▲, 1 mM) and detected by Congo red staining at the indicated time points. Addition of PBS alone (solvent for EGCG) served as negative control (τ). (B) Preformed PAP248-286-derived fibrils (SEVI) were treated with increasing concentrations of EGCG (▲, 1 mM; Δ, 2 mM; □, 4 mM; ○, 8 mM; and •, 10 mM) for 48 h and analyzed as before. Reactions containing EGCG alone (■, 10 mM) or PBS alone (τ) served as controls. (C) HIV-1 particles (X4-strain NL4/3) were preincubated with or without the indicated concentrations of SEVI. Subsequently, Jurkat 1G5 luciferase indicator cells were exposed to the respective virus/SEVI mixtures for 5 h. At 24 h after infection relative light units per second (RLU/s) were determined. Experiments were performed in triplicates. (D) Cellular metabolic activity was tested in uninfected Jurkat 1G5 cells by alamarBlue assay after 5 h of exposure to the indicated concentrations of SEVI. (E) Viabilities of uninfected Jurkat 1G5 cells were determined after 5 h exposure to the indicated EGCG concentrations as before.

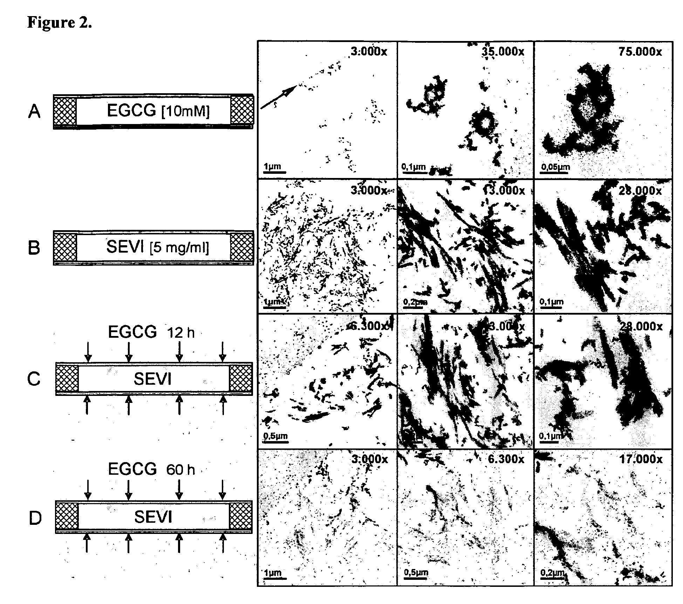

**Figure 2****. TEM-analysis of EGCG-treated SEVI in a closed system.** Corresponding images are arranged side by side. (A) Dissolved EGCG is shown inside of the ultrathin sectioned microtubes and at different magnifications. The overview (3.000x magnification) shows the interface between the tube wall (arrow) and the tube's lumen filled with EGCG. EGCG aggregates are organized in different patterns (35.000x and 75.000x magnification). (B) The SEVI solution shows a high density of SEVI-specific amyloid fibrils (3.000x magnification). Due to the embedding angle the fibrils often run out of the section plane (see 28.000x magnification). Nevertheless, their length differs between 30 and 90 nm. (C) Appearance of SEVI after 12 h of incubation in EGCG. The small EGCG aggregates diffused from the outside through the porous wall (6.300x magnification) into the lumen of the tube covering the surface of the fibrils. (D) Degradation of the majority of SEVI after 60 h of incubation in 10 mM EGCG.

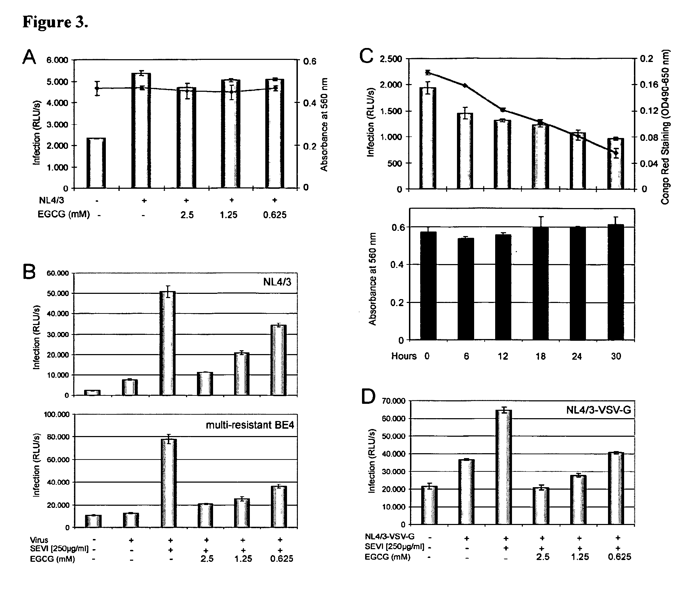

**Figure 3****. The green tea catechin EGCG inhibits SEVI-mediated HIV-infection.** (A) Jurkat 1G5 cells were infected with HIV-1 NL4/3 in presence of the indicated concentrations of EGCG. The rate of infection was monitored by measuring the luciferase activity in the respective cell cultures (indicated as RLU/s) and cell viabilities were determined by alamarBlue assay (red graph) 24 hours after infection. (B) 1G5 cells were infected with HIV-1 NL4/3 or the multiple antiretroviral drug-resistant virus isolate BE4 without and with 50 µg SEVI and the indicated concentrations of EGCG and luciferase activity was measured 24 h after infection as before. (C) Constant amounts of EGCG (2.5 mM) and SEVI (5 mg/ml) were incubated for the indicated time periods. The degradation of SEVI in each reaction was monitored by Congo red staining (blue graph). Subsequently, equal aliquots of each reaction were used to infect 1G5 cells with HIV-1 NL4/3. The respective cultures were assayed as before. Luciferase activities are shown in the upper panel. Metabolic activities are depicted in the lower panel. (D) Infection of 1G5 cells using VSV-G pseudotyed NL4/3 virions in presence and absence of the indicated concentrations of SEVI and EGCG. Cell cultures were assayed as before.

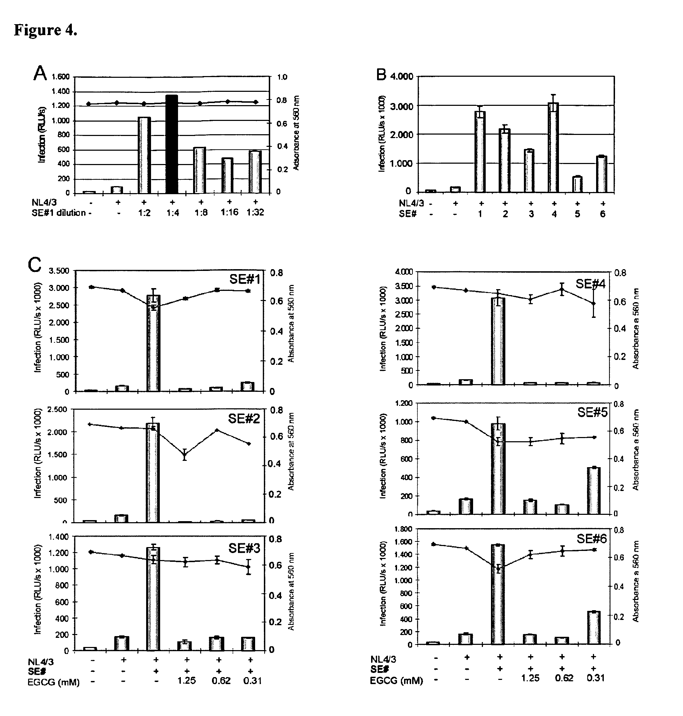

**Figure 4****. EGCG abrogates semen-enhanced virus infection.** (A) TZM-bl cells were infected with replication competent HIV-1 NL4/3 in the absence or presence of the indicated dilutions of a randomly selected human semen sample (SE#1). Non-infected cells and infected SE-untreated cells served as controls. Infectivity and cell viability (red graph) was determined as before. (B) Six different semen samples (SE#1-SE#6), diluted 1:4 4 in PBS, were analyzed in parallel as before. Experiments were performed in triplicates. (C) NL4/3-infections of TZM-bl cell cultures, which were exposed to individual SE samples #1 to #6 (1:4 dilution) in presence or absence of the indicated EGCG concentrations. Luciferase activity and cell viability (red graph) was determined at 24 h post infection as before. Experiments were performed in triplicates.

### EXAMPLES

### RESULTS AND DISCUSSION

### 1. Effects of the green tea catechin EGCG on PAP-derived amyloid fibrils

The capacity of a synthetic PAP248-286 peptide to form amyloid fibrils was analyzed in presence or absence of various concentrations of EGCG. Fibril formation of the freshly dissolved peptide (1 mg/ml and 5 mg/ml in PBS) was initiated by agitation at 37°C and monitored over time by Congo red staining, a standard method to detect amyloid fibrils (17). In agreement with previous data (4), fibril formation was easily observed at both peptide concentrations in absence of EGCG (Fig. 1 A). Further inspection of the data revealed, that EGCG interfered with fibrillogenesis in a dose-dependent fashion. The ratio of PAP248-286 to EGCG clearly affected the inhibitory capacity of EGCG in these experiments (Fig. 1A, compare upper and lower panel). Depending on the peptide concentration, high inhibitor levels (10 and 20 mM EGCG) completely abrogated the formation of fibrils. Furthermore, when EGCG was present in the reaction at lower concentrations (i.e. 1 mM and 5 mM) newly formed fibrils were apparently degraded over time (Fig. 1A).

To analyze this effect further, we next exposed preformed PAP248-286 fibrils, which were generated in absence of inhibitor, to various concentrations of EGCG. Again, the status of fibrillogenesis was determined over time by Congo red staining. As shown in figure 1 B, SEVI (5 mg/ml) was highly stable over a time-period of 48 hours. Obviously, fibril formation did even slightly increase within the initial 3 hours of incubation. In sharp contrast, however, additon of EGCG to the preformed amyloid fibrils apparently triggered their degradation, which was again observed in a dose-dependent manner. Control reactions, in which PBS only (solvent for EGCG) or EGCG only (10 mM) was analyzed, did, as expected, not score in this experiment.

We next wanted to confirm that our SEVI samples are indeed able to promote HIV-1 infection. For this, various concentrations of SEVI were preincubated with HIV-1 strain NL4/3 particles for 20 min in a small volume. Subsequently, this virus/SEVI mixture was used to infect Jurkat 1G5 cells, which contain a stably integrated HIV-1 long terminal repeat (LTR)-luciferase construct (18). Thus, infectability of these T cells can be easily quantitated by measuring luciferase activity. At 24 hours post infection the respective analyses revealed the expected SEVI-mediated enhancement of HIV-1 infection, clearly reaching an optimum in these cells at a SEVI concentration of 250 µg/ml (Fig. 1 C). The monitoring of cellular metabolic activity, as measured by alamarBlue assay, demonstrated that SEVI did not induce any detectable toxic effect on cellular viability in Jurkat 1G5 cells at these drug concentrations (Fig. 1 D).

Despite the fact that the polyphenol EGCG is a major constituent of green tea (16) and is therefore generally recognized as safe for human consumption, potential undesired effects of EGCG on the viability of uninfected 1G5 cells were next analyzed. As shown in Figure 1 E, even at 2.5 mM, the highest concentration tested, did EGCG not induce any deleterious effect in these cells.

To more directly monitor the action of EGCG on SEVI, we next performed analyses at the ultrastructural level using an ultrathin sectioning technique in combination with transmission electron microscopy (TEM). Solutions of SEVI or EGCG were encapsulated in capillary microtubes (200 µm diameter; depicted in figure 2), fixed in situ, dehydraded and embedded for subsequent staining of ultrathin sections and micrograph aquisition. As shown, a 10 mM solution of the polyphenol EGCG (mol wt 458.37) formed small aggregates, while significant larger typical fibrillary structures were observed in case of SEVI at a concentration of 5 mg/ml (Fig. 1 A and B, respectively). To next visualize the interaction between SEVI and EGCG, sealed microtubes filled with dissolved SEVI fibrils were placed into a chamber filled with a solution of 10 mM EGCG and subsequently incubated over time, allowing the EGCG molecules to diffuse from the outside into the lumen of the tube. After incubation for 12 and 60 hours the tubes were fixed and samples were processed as before. Obviously, the SEVI fibrils remained largely unaffected by EGCG after an incubation period of 12 hours, although EGCG aggregates already covered the surface of the SEVI fibrils, which is particularly apparent at larger magnification (Fig. 2 C). In sharp contrast, however, after incubation for 60 hours all SEVI-specific amyloid fibrils were extensively degraded (Fig. 2 D), as visualized by the low contrast of the SEVI residues, directly confirming the data obtained before by Congo red staining. Thus, the combined experiments demonstrated that the polyphenol EGCG targets PAP248-286-derived amyloid fibrils.

Clearly, the exact molecular mechanism of how EGCG degrades SEVI remains to be elucidated. Recently, experimental evidence has been presented indicating that EGCG inhibits amyloid formation of α-synuclein and amyloid-β by redirecting aggregation-prone molecules into alternative oligomers that do not contribute to plaque formation, and also prevents the seeded aggregation of both polypeptides (13). It is therefore conceivable that EGCG may also exert similar inhibitory effects on the fibrillogenesis of SEVI-specific aggregates.

### 2. EGCG inhibits the activity of PAP248-286-specific amyloid fibrils

It has been earlier reported that EGCG interferes with HIV-1 replication (19, 20) and various antiviral mechanisms of EGCG had been suggested, including inhibition of gp120-CD4 interaction (21, 22) and direct blocking of envelope gp41-mediated membrane fusion (23). However, the reported inhibitory potencies were rather modest (as compared to standard antiretroviral therapy) and some data were solely based on in vitro experiments using recombinant proteins. Nonetheless we next wanted to analyze the potential antiviral effect of EGCG in Jurkat 1G5 cells in absence of SEVI. As shown in figure 3 A, even at 2.5 mM no significant antiviral EGCG effects on de novo infection were observed in HIV-1 NL4/3 infected cells. By considering the results of the cell viability tests performed before (Fig. 1 D and E), it was not surprising that no cellular toxicity was detectable in these cell cultures (Fig. 3 A; red graph).

Since EGCG apparently degrades SEVI, we next wanted to test whether EGCG indeed inhibits the infectivity enhancing action of these amyloid fibrillary aggregates. We therefore again infected 1G5 cells with either the HIV-1 NL4/3 strain or the multi-resistant BE4 isolate and monitored infectability by measuring luciferase activity as before. HIV-1 BE4 is a highly active antiretroviral therapy (HAART)-resistant virus with high-level resistance to multiple inhibitors of viral protease and reverse transcriptase (24). As before, prior to infection the respective input viruses were preincubated with SEVI (250 µg/ml) together with the concentrations of EGCG analyzed before to be non-toxic in these cells (see Fig. 1 E). These experiments confirmed again the infectivity enhancing properties of SEVI and demonstrated, that EGCG is indeed capable to effectively counteract the activity of PAP248-286-derived fibrils in a dose-dependent fashion (Fig. 3 B). In fact, at the highest concentration tested (2.5 mM) EGCG almost completely abrogated SEVI-specific boosting of virus infection. Moreover, this inhibitory effect was observed independent from the virus genotype or phenotype (i.e. strain NL4/3 versus HAART-resistant BE4).

To further verify that the inhibitory activity of EGCG on HIV-1 infection in our experiments was primarily based on SEVI degradation, we now performed a time lapse experiment, in which the amount of EGCG (2.5 mM) and SEVI (5 mg/ml) were kept constant. As shown in figure 3C, increasing incubation times resulted in the gradual degradation of SEVI as measured by Congo red staining in each reaction (upper panel; blue graph). Importantly, a strictly linear correlation of the HIV-1 infectivity boosting capacity of each sample with the amount of SEVI available in the respective sample was observed (Fig. 3C, upper panel). In agreement with the data raised before, no cellular toxicity was observed in the corresponding cell cultures (Fig. 3C, lower panel).

Finally, we utilized vesicular stomatitis virus glycoprotein (VSV-G) pseudotyped HIV-1 particles in the next series of experiments. Pseudotyped viral particles have a broad host range, since VSV-G presumably interacts with ubiquitous membrane lipids and possibly other components of lipid bilayers (25). Thus, by using VSV-G pseudotypes we were able to bypass the otherwise strict requirement of the HIV-1 glycoproteins (gp120/gp41) for host cell infection. As shown in Figure 3 D, SEVI also enhanced the infectivity of these pseudotyped virions and this effect was again abrogated by EGCG, although to a slightly higher extent as seen in wildtype viruses. This effect was repeatedly observed and may indicate additional SEVI-unrelated inhibitory effects of EGCG on pseudotyped virions.

In sum, these data demonstrated that SEVI is a rather general enhancer of virus infectivity, which agrees with its postulated mode of action (6) and, importantly, EGCG antagonizes its activity.

### 3. EGCG interferes with semen-mediated enhancement of HIV-1 infection

To this point we demonstrated that EGCG can efficiently suppress the action of SEVI, that has been generated in vitro. However, any potential therapeutic application of EGCG (or of related compounds) depends on its ability to inactivate the infectivity-enhancing capacity of human semen (SE). Thus, to more closely mirror the in vivo situation we now included different human SE samples into our analyses.

It has been recently reported that addition of undiluted human SE might be toxic to cultured cells (4). Thus, we first investigated the infectivity enhancing qualities of a serial dilution of an at random selected human SE sample (SE#1). Infection of TZM-bl cells (26) with HIV-1 NL4/3 demonstrated that none of the SE#1 dilutions (1:2 to 1:32 in PBS/antibiotics) induced cellular toxicity (Fig. 4 A, red graph) and, that application of particularly the 1:4 dilution resulted in maximal infection of these cells (Fig. 4 A). Subsequently, additional 1:4 dilutions of independent SE samples (SE#2-6) were compared side-by-side with SE#1 using the same experimental set-up (Fig. 4 B). Inspection of the corresponding results indicated that individual human SE samples may considerably vary with respect to their HIV-1 infectivity boosting properties (e.g. compare SE#4 and SE#5 in Fig. 4 B). Nevertheless, the majority of these random samples did significantly increase virus infection as compared to the control experiment in which SE was omitted.

Thereupon, the effect of EGCG on preventing semen-mediated enhancement of HIV-1 infection was tested on all SE samples as before. This series of experiments demonstrated that the SEVI-inhibitor EGCG also efficiently abrogated the infectivity enhancing properties of human SE in absence of significant cellular toxicity (Fig. 4 C). In general it appeared that this inhibitory effect required lower drug concentrations as in the experiments analyzing *in vitro* generated SEVI (compare Fig. 3B and Fig. 4 C), and that also some differences between the various SE samples exist with respect to EGCG sensitivity (e.g. compare SE#4 and SE#5), which may reflect differences in the amount of infectivity-enhancing fibrils that are present in the individual samples.

The combined data presented in this study suggest that the polyphenol EGCG targets PAP248-286-derived amyloid fibrils for degradation, thereby efficiently abrogating their HIV-1 infectivity-enhancing properties. Previous studies on the anti-HIV effects of EGCG focussed on systemic application of this compound (20, 25). In contrast, our data argue in favour of the administration of EGCG (or functionally related drugs) as a topical agent, possibly as a supplement of antiretroviral microbicides. Thereby their antiretroviral potency may be improved due to EGCG-mediated compensation of the infectivity boosting qualities of human semen. The fact that EGCG is very stable in acidic solution (27), a condition similar to the vaginal environment, is further supporting this notion. The recent finding that EGCG also damages herpes simplex virions (28), an activity that under certain circumstances might also apply to HIV-1 (20), may even provide an additional antiviral benefit with respect to the usage of EGCG as a component of future microbicides. Taken together, the polyphenol EGCG, a dietary supplement from green tea, may be a valuable and cost-efficient inhibitor of the semen-mediated enhancement of virus infection and hence, of sexual transmission of HIV-1.

### MATERIALS AND METHODS

**Reagents.** The major catechin in green tea, epigallocatechin-3-gallate (EGCG ((-)EGCG); mol wt 458.37), was obtained from Sigma-Aldrich and a stock solution of 100 mM was prepared in PBS. Aliquots of various dilutions were stored at -20°C.

**Amyloid fibril formation by PAP248-286 and photometric detection.** Synthetic peptides, corresponding to PAP (EMBL accession #: AAB60640) amino acid residues 248-286 (PAP248-286), were obtained from Davids Biotechnologie GmbH (Regensburg) and Bachem AG (Basel). Lyophilized peptides were resuspended in PBS at a stock concentration of 10 mg/ml and aliquots were stored at -20°C. Fibril formation by the fresh dissolved peptide (1 mg/ml or 5 mg/ml) was initiated by agitation at 37°C and 1.200 rpm for 2-3 days (i.e. until the solution became turbid) using an Eppendorf Thermomixer. Formation of amyloid structures was routinely monitored by mixing 5 µl aliquots of the respective reaction batch with 100 µl Congo red solution (20 µg/ml in PBS, Sigma). The solution was incubated for 10 min at ambient temperature and centrifuged for 5 min at 14.000 rpm in an Eppendorf microfuge. Supernatants were discarded and the pellets were dissolved in 100 µl DMSO and fibril formation was determined at OD₄₉₀₋₆₅₀ nm using an ELISA reader.

**Treatment of semen.** Semen (SE) samples were donated by different lab-members, diluted with equal volumes of PBS containing 100 U/ml penicillin, 100 µg/ml streptomycin and 50 µg/ml gentamycin (Gibco) and stored at -20°C.

**Analysis of cellular toxicity**. Cell viability was analyzed by measuring cellular metabolic activity using the alamarBlue redox indicator (Serotec), in accordance with the manufacturer's protocol.

**Cell cultures and HIV-infection experiments**. The suspension cell line 1G5 was cultured in RPMI 1640 medium containing 10% FCS (PanSystems GmbH) and antibiotics (penicillin and streptomycin). 1G5 cells are Jurkat derivatives containing a stably integrated HIV-LTR-luciferase construct (18). For HIV-infection, 10⁶ cells were incubated in a total volume of 200 µl at 37° C with 50 ng of p24 antigen of either HIV-1 NL4/3 or the multi-resistant HIV isolate BE4 (24), 50 µg of PAP248-286-derived amyloid fibrils (SEVI) and various concentrations of EGCG. Control experiments omitted the input virus, SEVI or EGCG. After a 5 h incubation period, cells were pelleted, washed once in PBS and resuspended in 1 ml medium. At 24 h post-infection, cell cultures were divided. One half was used to analyze cell viability. The cells of the remaining half were lysed in 100 µl passive lysis buffer and luciferase activity was measured as relative light units per second (RLU/s) according to the manufacturer's protocol (Promega). The infectivity enhancing properties of independent human semen (SE) samples were analyzed in the adherent TZM-bl cell line, which allows the quantification of HIV-infection either via an integrated Tat-responsive β-galactosidase or luciferase reporter expression cassette (26). For this, 200 µl of an 1:4 dilution of independent SE-samples in PBS were mixed with 50 ng of p24 antigen either of HIV-1 NL4/3 or VSV-G pseudotyped NL4/3Δenv (29) together with various concentrations of EGCG and incubated in an Eppendorf thermomixer at 37° C and 800 rpm for 20 min.

Thereafter, 30 µl of the mixtures were added to 10⁴ cells TZM-bl cells in a volume of 100 µl and incubated at 37° C for 3 h, washed once, and further cultured in 200 µl DMEM (10% FCS, penicillin, streptomycin) in a 96-well flat-bottom plate. At 24 h post infection, virus infectivity and cell viability was determined. As before, controls omitting input virus, SE or EGCG were also included into the experimental design. VSV-G pseudotypes were produced as described before in detail (29).

**Electron microscopy**. For ultrastructural analysis of the dissolved components in the transmission electron microscope (TEM) the solutions of SEVI (5 mg/ml) and EGCG (10 mM) were encapsulated in capillary microtubes and processed for ultrathin sectioning technique as already described in principle and detail (30). The microtubes (200 µm diameter) were filled with the solutions by capillary attraction and mechanically sealed at both ends by a scalpel. The dissolved components within the tubes were fixed in situ from outside through the highly porous tube walls (MWCO:10 kD) by successive immersion into 2.5% glutaraldehyde, 1% uranyl acetate and 1% OsO₄ in PBS for 30 min each, followed by dehydration in graded series of ethanol. For ultrathin sectioning the microtubes were embedded in EPON (Carl Roth GmbH + Co) resin. Sections were counter-stained with 2% uranyl acetate and lead citrate. Electron micrographs were acquired with a Philips CM 120 TEM at 80 kV using a Gatan Multiscan 794 Camera.

To illustrate the interaction between SEVI and EGCG, sealed microtubes filled with dissolved SEVI were incubated into a EGCG solution. After defined reaction times the tubes were fixed and processed as described above.

### REFERENCES

1. 2007. AIDS epidemic update. *UNAIDS.*
2. Shattock, R.J. and J.P. Moore. 2003. Inhibiting sexual transmission of HIV-1 infection. Nat.Rev.Microbiol. 1:25-34.
3. Klasse, P.J., R. Shattock, and J.P. Moore. 2008. Antiretroviral drug-based microbicides to prevent HIV-1 sexual transmission. Annu.Rev.Med. 59:455-471.
4. Münch, J., E. Rücker, L. Ständker, K. Adermann, C. Goffinet, M. Schindler, S. Wildum, R. Chinnadurai, D. Rajan, A. Specht, G. Gimenez-Gallego, P.C. Sanchez, D.M. Fowler, A. Koulov, J.W. Kelly, W. Mothes, J.C. Grivel, L. Margolis, O.T. Keppler, W.G. Forssmann, and F. Kirchhoff. 2007. Semen-derived amyloid fibrils drastically enhance HIV infection. Cell 131:1059-1071.
5. Rönnberg, L., P. Vihko, E. Sajanti, and R. Vihko. 1981. Clomiphene citrate administration to normogonadotropic subfertile men: blood hormone changes and activation of acid phosphatase in seminal fluid. Int.J.Androl 4:372-378.
6. Roan, N.R. and W.C. Greene. 2007. A seminal finding for understanding HIV transmission. Cell 131:1044-1046.
7. Haase, A.T. 2005. Perils at mucosal front lines for HIV and SIV and their hosts. Nat.Rev.Immunol. 5:783-792.
8. Grant, R.M., D. Hamer, T. Hope, R. Johnston, J. Lange, M.M. Lederman, J. Lieberman, C.J. Miller, J.P. Moore, D.E. Mosier, D.D. Richman, R.T. Schooley, M.S. Springer, R.S. Veazey, and M.A. Wainberg. 2008. Whither or wither microbicides? Science 321:532-534.
9. Dobson, C.M. 2003. Protein folding and misfolding. Nature 426:884-890.
10. Rochet, J.C. and P.T. Lansbury, Jr. 2000. Amyloid fibrillogenesis: themes and variations. Curr.Opin.Struct.Biol. 10:60-68.
11. Taylor, J.P., J. Hardy, and K.H. Fischbeck. 2002. Toxic proteins in neurodegenerative disease. Science 296:1991-1995.
12. Sacchettini, J.C. and J.W. Kelly. 2002. Therapeutic strategies for human amyloid diseases. Nat.Rev.Drug Discov. 1:267-275.
13. Ehrnhoefer, D.E., J. Bieschke, A. Boeddrich, M. Herbst, L. Masino, R. Lurz, S. Engemann, A. Pastore, and E.E. Wanker. 2008. EGCG redirects amyloidogenic polypeptides into unstructured, off-pathway oligomers. Nat.Struct.Mol.Biol. 15:558-566.
14. Yang, C.S., P. Maliakal, and X. Meng. 2002. Inhibition of carcinogenesis by tea. Annu.Rev.Pharmacol.Toxicol. 42:25-54.
15. Nance, C.L. and W.T. Shearer. 2003. Is green tea good for HIV-1 infection? J.Allergy Clin.Immunol. 112:851-853.
16. Cabrera, C., R. Artacho, and R. Gimenez. 2006. Beneficial effects of green tea--a review. J.Am.Coll.Nutr. 25:79-99.
17. Frid, P., S.V. Anisimov, and N. Popovic. 2007. Congo red and protein aggregation in neurodegenerative diseases. Brain Res.Rev. 53:135-160.
18. Aguilar-Cordova, E., J. Chinen, L. Donehower, D.E. Lewis, and J.W. Belmont. 1994. A sensitive reporter cell line for HIV-1 tat activity, HIV-1 inhibitors, and T cell activation effects. AIDS Res.Hum.Retroviruses 10:295-301.
19. Fassina, G., A. Buffa, R. Benelli, O.E. Varnier, D.M. Noonan, and A. Albini. 2002. Polyphenolic antioxidant (-)-epigallocatechin-3-gallate from green tea as a candidate anti-HIV agent. AIDS 16:939-941.
20. Yamaguchi, K., M. Honda, H. Ikigai, Y. Hara, and T. Shimamura. 2002. Inhibitory effects of (-)-epigallocatechin gallate on the life cycle of human immunodeficiency virus type 1 (HIV-1). Antiviral Res. 53:19-34.
21. Kawai, K., N.H. Tsuno, J. Kitayama, Y. Okaji, K. Yazawa, M. Asakage, N. Hori, T. Watanabe, K. Takahashi, and H. Nagawa. 2003. Epigallocatechin gallate, the main component of tea polyphenol, binds to CD4 and interferes with gp120 binding. J.Allergy Clin.Immunol. 112:951-957.
22. Williamson, M.P., T.G. McCormick, C.L. Nance, and W.T. Shearer. 2006. Epigallocatechin gallate, the main polyphenol in green tea, binds to the T-cell receptor, CD4: Potential for HIV-1 therapy. J.Allergy Clin.Immunol. 118:1369-1374.
23. Liu, S., H. Lu, Q. Zhao, Y. He, J. Niu, A.K. Debnath, S. Wu, and S. Jiang. 2005. Theaflavin derivatives in black tea and catechin derivatives in green tea inhibit HIV-1 entry by targeting gp41. Biochim.Biophys.Acta 1723:270-281.
24. Hauber, I., D. Bevec, J. Heukeshoven, F. Krätzer, F. Horn, A. Choidas, T. Harrer, and J. Hauber. 2005. Identification of cellular deoxyhypusine synthase as a novel target for antiretroviral therapy. J.Clin.Invest 115:76-85.
25. Cronin, J., X.Y. Zhang, and J. Reiser. 2005. Altering the tropism of lentiviral vectors through pseudotyping. Curr.Gene Ther. 5:387-398.
26. Derdeyn, C.A., J.M. Decker, J.N. Sfakianos, X. Wu, W.A. O'Brien, L. Ratner, J.C. Kappes, G.M. Shaw, and E. Hunter. 2000. Sensitivity of human immunodeficiency virus type 1 to the fusion inhibitor T-20 is modulated by coreceptor specificity defined by the V3 loop of gp120. J. Virol. 74:8358-8367.
27. Zhu, Q.Y., A. Zhang, D. Tsang, Y. Huang, and Z.-Y. Chen. 2008. Stability of green tea catechins. J.Agric.Food Chem. 45:4624-4628.
28. Isaacs, C.E., G.Y. Wen, W. Xu, J.H. Jia, L. Rohan, C. Corbo, M. Di, V, E.C. Jenkins, Jr., and S. Hillier. 2008. Epigallocatechin gallate inactivates clinical isolates of herpes simplex virus. Antimicrob.Agents Chemother. 52:962-970.
29. Sarkar, I., I. Hauber, J. Hauber, and F. Buchholz. 2007. HIV-1 proviral DNA excision using an evolved recombinase. Science 316:1912-1915.
30. Hohenberg, H., K. Mannweiler, and M. Müller. 1994. Highpressure freezing of cell suspensions in cellulose capillary tubes. J.Microsc. 175:34-43.
31. WO 2005/077343
32. US 2002/0151506
33. US 2002/0086067
34. US 5,795,911
35. US 5,968,973

## Claims

1. Pharmaceutical composition for topical administration for prevention of sexual transmission of viral infection, wherein the composition comprises at least one compound from the class of catechins.

2. Use of at least one compound from the class of catechins for the preparation of a pharmaceutical composition formulated for topical administration for prevention of sexual transmission of viral infection.

3. Composition according to claim 1 or use according to claim 2, wherein the compound is selected from the group consisting of catechin (C), catechin-gallate (CG), gallocatechin (GC), gallocatechin-gallate (GCG), epicatechin (EC), epicatechin-gallate (ECG), epigallocatechin (EGC), epigallocatechin-gallate (EGCG), and derivatives thereof which are capable of disrupting preformed SEVI (semen-derived enhancer of virus infection) fibrils.

4. Composition or use according to claim 3, wherein the compound is epigallocatechin-gallate (EGCG).

5. Composition or use according to to one of the preceding claims, wherein the compound is the (-) epimer.

6. Composition or use according to one of the preceding claims, wherein the compound is derived from tea, e.g., green tea, in particular from tea leaves or an extract thereof.

7. Composition or use according to one of the preceding claims, wherein the composition comprises green tea (*Camellia sinensis*) extract.

8. Composition or use according to one of the preceding claims, wherein the infection is retroviral infection and/or infection with a DNA containing virus.

9. Composition or use according to one of the preceding claims, wherein the infection is HIV infection, HTLV infection, HPV infection, Hepatitis B infection, Hepatitis C infection, human Herpesvirus 8 infection and/or Herpes-simplex virus infection.

10. Composition or use according to one of the preceding claims, wherein the composition is for topical administration to the vagina of a woman before and/or after sexual intercourse.

11. Composition or use according to one of the preceding claims, wherein the composition is formulated as an ointment, cream, gel, suppository, liquid for vaginal irrigation, intravaginal ring or concentrate for preparation of the same.

12. Composition or use according to one of the preceding claims, wherein the composition is formulated to achieve intravaginal concentrations of the compound of about 0.1 mM or more, about 0.25 mM or more, about 0.5 mM or more, about 1 mM or more or about 2.5 mM or more.

13. Composition or use according to one of the preceding claims, wherein the composition comprises about 2 - 20 % by weight of the compound.

14. Method for preventing sexual transmission of viral infection, comprising topically administering a composition comprising at least one compound from the class of catechins.
